(19) Europäisches Patentamt
European Patent Office
Office européen des brevets

(11) Veröffentlichungsnummer: **0 058 236 B1**

# (12) EUROPÄISCHE PATENTSCHRIFT

(45) Veröffentlichungstag der Patentschrift: **25.04.84**

(51) Int. Cl.³: **C 07 C 69/16**, C 07 C 67/055

(21) Anmeldenummer: **81108837.6**

(22) Anmeldetag: **24.10.81**

(54) **Diacyloxyalkadiene und ihre Herstellung.**

(30) Priorität: **15.01.81 DE 3101002**

(43) Veröffentlichungstag der Anmeldung: **25.08.82 Patentblatt 82/34**

(45) Bekanntmachung des Hinweises auf die Patenterteilung: **25.04.84 Patentblatt 84/17**

(84) Benannte Vertragsstaaten: **BE CH DE FR GB IT LI**

(56) Entgegenhaltungen:
**EP - A - 0 001 593**
**FR - A - 2 366 256**

**TETRAHEDRON LETTERS, Nr. 33, 1978, Seiten 3063-3064, Pergamon Press Oxford, G.B. A.J. BLACKMAN et al.: "Flexilin and trifarin, terpene 1,4-diacetoxybuta-1,3-dienes from two caulerpa species (chlorophyta)**
**Houben-Weyl, Methoden der organischen Chemie, 4. Auflage, Band V/1b Seiten 663-665 (1972), Georg Thieme Verlag Stuttgart**

(73) Patentinhaber: **BASF Aktiengesellschaft, Carl-Bosch-Strasse 38, D-6700 Ludwigshafen (DE)**

(72) Erfinder: **Fischer, Rolf, Dr., Bergstrasse 98, D-6900 Heidelberg (DE)**
Erfinder: **Weitz, Hans-Martin, Dr., Auf dem Koeppel 40, D-6702 Bad Duerkheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Diacyloxyalkadiene und ihre Herstellung

Die Erfindung betrifft neue Diacyloxyalkadiene der im Anspruch 1 angegebenen Formel, die mindestens zwei nicht miteinander konjugierte Doppelbindungen enthalten und deren Acyloxygruppen in 1,4-Stellung zueinander stehen, und ein Verfahren zu ihrer Herstellung durch Umsetzung von aliphatischen Trienen, die zwei miteinander konjugierte und mindestens eine isolierte Doppelbindung enthalten, mit Carbonsäuren und Sauerstoff in Gegenwart von Katalysatoren gemäß dem Patentanspruch 2.

Die neuen Diacyloxyalkadiene haben die allgemeine Formel

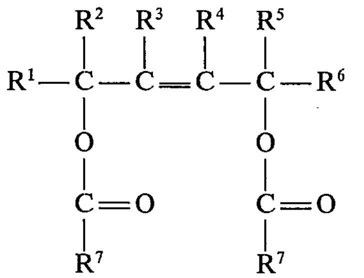

(I)

in der die Reste $R^1$ bis $R^5$ Wasserstoffatome oder Kohlenwasserstoffreste mit 1 bis 6 C-Atomen, $R^6$ ein Wasserstoffatom oder einen Kohlenwasserstoffrest mit 3 bis 9 C-Atomen und mindestens einer nicht-konjugierten Doppelbindung und $R^7$ ein Wasserstoffatom oder einen Alkylrest mit 1 bis 5 C-Atomen bedeuten, und für den Fall, daß $R^6$ ein Wasserstoffatom bedeutet, mindestens einer der Reste $R^1$ bis $R^5$ für einen $R^8-CH_2$-Rest steht, in dem $R^8$ einen Kohlenwasserstoffrest mit 2 bis 5 C-Atomen bedeutet, der mindestens eine nicht-konjugierte Doppelbindung enthält.

$R^1$ bis $R^5$ können für Wasserstoffatome oder Kohlenwasserstoffreste mit 1 bis 6 C-Atomen stehen. Die Kohlenwasserstoffreste sind vorzugsweise geradkettige Reste, die auch Doppelbindungen enthalten können, welche jedoch weder zueinander noch zu den Doppelbindungen in den Ausgangsstoffen der Formel II in Konjugation stehen. Kohlenwasserstoffreste dieser Art sind z. B. Alkyl, Alkenyl- oder Alkadienylreste, wie $CH_3$-, $C_2H_5$-, $C_3H_7$-, n-$C_4H_9$-, sek-Butyl-, i-Butyl-, tert-Butyl-, $CH_2=CH$-, $CH_3-CH=CH$-, $CH_3-CH=CH-CH_2$, $CH_3-C(CH_3)=CH$-, $CH_3-C(CH_3)=CH-CH_2$-, $CH_3-C(CH_3)=CH-CH_2-CH_2$-, $CH_2=CH-CH_2$-, $CH_2=C(CH_3)-CH_2$-, $CH_2=CH-C(CH_3)$-, $CH_2=CH-CH_2-CH_2$-, $CH_2=CH-CH_2-CH=CH$- oder $CH_2=CH-CH_2-CH=CH-CH_2$-Reste.

Der Kohlenwasserstoffrest $R^6$ weist 3 bis 9 C-Atome und mindestens eine nicht-konjugierte Doppelbindung auf. Reste dieser Art haben z. B. die Formel $-CH_2-C(CH_3)=CH_2$, $-CH_2-CH_2-C(CH_3)=CH_2$, $-CH_2-CH_2-CH=CH_2$, $-CH_2-C(CH_3)=CH-CH_3$, $-CH_2-CH=CH-(CH_2)_3-CH=CH_2$, $-CH_2-CH=CH_2$, $-CH_2-C(CH_3)=CH_2$, $-CH_2-CH=CH-CH_3$, $-CH_2-CH=C(CH_3)_2$, $-CH_2-CH=CH-CH_2-CH=CH_2$, $-CH_2-CH=CH-(CH_2)_2-CH=CH_2$oder$-CH_2-CH=CH-(CH_2)_4-CH=CH_2$.

Als Alkylreste $R^7$ kommen z. B. Methyl-, Ethyl-, Propyl- und Butylreste in Betracht.

Von besonderem technischem Interesse sind die Diacyloxydialkadiene der Formel I, die insgesamt zwei oder drei nicht-konjugierten Doppelbindungen enthalten und in der $R^7$ ein Wasserstoffatom oder eine Alkylgruppe mit 1 bis 3 C-Atomen bedeutet.

Die neuen Diacyloxyalkadiene der Formel I werden dadurch hergestellt, daß man aliphatische Triene der Formel

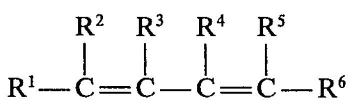

(II)

in der die Reste $R^1$ bis $R^6$ die obengenannte Bedeutung haben, in Gegenwart von Trägerkatalysatoren, die Palladium oder Platin, und außerdem noch Kupfer und/oder Tellur enthalten, und von Sauerstoff mit Carbonsäuren der Formel $R^7-COOH$, in der $R^7$ die obengenannte Bedeutung hat, bei Temperaturen von 70 bis 180° C behandelt.

Die Reaktion kann für den Fall der Umsetzung von 1,3,7-Octatrien mit Sauerstoff und Essigsäure durch die folgenden Formeln wiedergegeben werden:

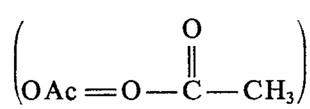

$$CH_2{=}CH{-}CH{=}CH{-}CH_2{-}CH_2{-}CH{=}CH_2 + 2\,CH_3{-}COOH + \frac{1}{2}\,O_2$$

$$\downarrow$$

$$\underset{\displaystyle OAc}{\underset{|}{CH_2}}{-}CH{=}CH{-}\underset{\displaystyle OAc}{\underset{|}{CH}}{-}CH_2{-}CH_2{-}CH{=}CH_2 + H_2O$$

Es ist bekannt, daß sich 1,3-Butadien, das durch Alkyl- oder Acyloxygruppen substituiert sein kann, mit Sauerstoff und Carbonsäuren in Gegenwart von Palladium oder Platin enthaltenden Katalysatoren zu 2-Buten-1,4-diol-diestern und 1-Buten-3,4-diol-diestern umsetzen läßt. Hierfür können homogen gelöste Katalysatoren oder Trägerkatalysatoren verwendet werden. So erhält man beispielsweise nach den Angaben der GB-PS 1 138 366 durch Umsetzung von Butadien mit Eisessig in Gegenwart von Palladiumchlorid, Kupferacetat und Lithiumacetat Gemische aus 1,4-Diacetoxy-2-buten und 3,4-Diacetoxy-1-buten im Molverhältnis 1 : 1. In ähnlicher Weise wird 1,3-Butadien nach der GB-PS 1 277 837 mit Sauerstoff in Gegenwart von in Eisessig gelöstem Palladiumacetat, Kupferacetat und Kaliumacetat zu 1,4-Diacetoxy-2-buten umgesetzt.

In der DE-PS 2 217 452 wird ein Verfahren zur Acetoxilierung von Butadien beschrieben, bei dem man Palladium-Trägerkatalysatoren verwendet, die zusätzlich Antimon, Bismut, Tellur oder Selen enthalten. Nach der DE-OS 2 820 519 verwendet man Trägerkatalysatoren, die Palladium und Kupfer in Form einer intermetallischen Verbindung enthalten. Die DE-OS 2 417 558 beschreibt Platin-Trägerkatalysatoren, die zusätzlich eines der Elemente Phosphor, Arsen, Antimon, Selen oder Tellur enthalten. Bei der Umsetzung von Butadien mit Sauerstoff und Essigsäure (»Acetoxilierung«) entstehen in Gegenwart der genannten Katalysatoren ganz überwiegend cis- und trans-2-Buten-1,4-dioldiacetate. 1-Buten-3,4-diolmono- und 1-Buten-3,4-dioldiacetate werden in untergeordnetem Maße gebildet. Aus der EP-A1-0 001 593 ist bekannt, daß man für die Herstellung von 2-Buten-1,4-dioldiacetat aus Butadien, Sauerstoff und Essigsäure ohne Nachteil auch $C_4$-Schnitte mit wechselnden Gehalten an Olefinen heranziehen kann.

Es ist weiterhin bekannt, daß die Umsetzung von Ethylen in Anwesenheit von Palladiumacetat, Kupferchlorid, Alkaliacetaten und Essigsäure Gemische aus 1,2-Diacetoxyethan und 1-Hydroxy-2-acetoxyethan und 1-Chlor-2-acetoxyethan liefert (P. M. Henry, J. Org. Chem. 32, 2575 [1967] und daß Palladium, Platin und ihre Verbindungen olefinische Doppelbindungen innerhalb von Kohlenstoffketten zu verschieben vermögen (Houben-Weyl Methoden der organischen Chemie, 4. Auflage, Band V/1b S. 663—665 [1972]).

Aufgrund dieses Standes der Technik mußt erwartet werden, daß bei dem erfindungsgemäßen Verfahren eine Anlagerung von Acyloxygruppen an die isolierten Doppelbindungen oder aber eine Verschiebung der isolierten Doppelbindungen erfolgt. Daß dies nicht eintritt ist überraschend. Auch liefert das erfindungsgemäße Verfahren die neuen Diacyloxyalkadiene auf besonders vorteilhaftem Wege. Eine mögliche Alternative, nämlich die an sich bekannte Anlagerung von Brom an 1,3-Diene, wie Isopren und die anschließende Umsetzung von 1,4-Dibrom-2-alkenen mit Alkaliacetaten zum Zwecke der Herstellung von 1,4-Diacetoxyverbindungen (A. F. Shepard, J. R. Johnson, J. Amer. Chem. Soc. 54, 4388 [1932]) würde auf die Triene der Formel II angewendet, zu einer Bromaddition an der isolierten Doppelbindung führen.

Als Ausgangsstoffe der Formel II geeignete Triene seien z. B. 1,3,6-Octatrien, 1,3,7-Octatrien, 2,6- und 2,7-Dimethyl-1,3,7-octatrien, 3,7-Dimethyl-1,3,6-octatrien (Ocimen) , Myrcen, 1,3,6.10-Dodecatetraen und 1,5,7.10.15-Hexadecapentaen genannt. Man kann die Olefine der Formel II für sich oder als Mischungen, die z. B. auch noch andere Kohlenwasserstoffe, wie Monoolefine und Paraffinkohlenwasserstoffe enthalten, einsetzen.

Die genannten Ausgangsverbindungen lassen sich beispielsweise durch Dimerisierung und Trimerisierung von 1,3-Dienen herstellen. So sind 1,3,6- und 1,3,7-Octatrien durch Dimerisierung von Butadien in Gegenwart von Nickelkatalysatoren (DE-OS 1 443 442), 2,6- und 2,7-Dimethyl-1,3,7-octatrien durch Dimerisierung von Isopren in Gegenwart von Palladiumverbindungen (H. Yagi et al., Synthesis 1977, 334), 1,3,6.10-Dodecatetraen durch Trimerisierung von Butadien (D. Medima, R. van Helden, Rec. Trav. Chim 90, 324 [1971]) und 1.5.7.10.15-Hexadecapentaen durch Dimerisierung von 1,3,7-Octatrien (W. Keim, H. Chung, J. Org. Chem. 37, 947 [1972]) zugänglich.

Als Carbonsäuren der geeigneten Art kommen z. B. Ameisensäure, Essigsäure, Propionsäure, Buttersäuren oder Valeriansäuren in Betracht.

Als Katalysatoren sind Trägerkatalysatoren geeignet, die als aktive Bestandteile, welche auf dem Trägermaterial aufgebracht sind, Palladium oder Platin und Kupfer und/oder Tellur enthalten. Die Katalysatoren lassen sich in bekannter Weise, z. B. nach den Angaben der DE-PS 2 217 452, DE-OS 2 820 519 oder DE-OS 2 417 452, DE-OS 2 820 519 oder DE-OS 2 417 558 herstellen. Katalysatoren der genannten Art enthalten beispielsweise, bezogen auf das Katalysatorgewicht, 1 bis 10% Palladium oder Platin, 0,1 bis 30% Kupfer und/oder 0,01 bis 10% Tellur. Bevorzugt ist die Verwendung eines Trägerkatalysators, der je Grammatom Palladium oder Platin 0,01 bis 6, vorzugsweise 1 bis 3,5 Grammatome Kupfer und/oder 0,01 bis 1, vorzugsweise 0,01 bis 0,4 Grammatome Tellur enthält.

Die Gesamtmenge der auf den Träger aufgebrachten katalytisch wirksamen Metalle beträgt, bezogen auf den Trägerkatalysator, zweckmäßig z. B. 0,01 bis 30 Gew.-%. Größere und kleinere Konzentrationen sind allerdings auch möglich. Als Trägermaterial enthalten die Katalysatoren z. B. Aktivkohle, Bauxit, Bimsstein, Kieselgel, Kieselgur oder andere Formen der Kieselsäure, Magnesia, Ton oder Tonerde.

Man kann die katalytisch wirksamen Metalle z. B. auch ohne Trägermaterial anwenden, indem man sie in Form von Salzen im Reaktionsgemisch löst oder suspendiert.

Die Umsetzung zur Herstellung der Diacyloxyalkadiene nimmt man in an sich bekannter Weise in der Gas- oder Flüssigphase bei Temperaturen von 70 bis 180°C vor. In der Gasphase arbeitet man vorzugsweise bei 120 bis 150°C und in der Flüssigphase vorzugsweise bei 70 bis 110°C. Der Reaktionsdruck ist durch die Verfahrensweise gegeben und kann zwischen atmosphärischem Druck und z. B. 100 bar betragen. Das Verfahren kann chargenweise oder kontinuierlich z. B. mit fixiertem Bett, Wirbelbett oder Dreiphasenfließbett durchgeführt werden. Unumgesetzte Triene II können nach der Umsetzung zusammen mit der jeweiligen Carbonsäure aus dem Reaktionsgemisch abdestilliert und in Form dieses Gemisches wieder eingesetzt werden.

Die erfindungsgemäßen Diacyloxyalkadiene der Formel I sind wertvolle Zwischenprodukte. Die aus ihnen nach Hydrierung und Verseifung hervorgehenden substituierten 1,4-Diole können wie 1,4-Butandiol, 1,6-Hexandiol (Ullmanns Encyklopädie, Band 7, Seite 228)und 1,8-Octandiol (DE-OS 1 066 566) für die Herstellung von Polyurethanen, Estern und Weichmachern verwendet werden. Solche Diacyloxyalkadiene der Formel I, in denen $R^1$, $R^2$, $R^4$ und $R^5$ Wasserstoffatome, $R^3$ und $R^6$ bis $R^8$ Alkyl- und Alkenylreste und $R^7$ Alkylreste bedeuten, lassen sich mit Wasser in Gegenwart von Kationenaustauschern oder Mineralsäuren zu $\alpha,\beta$-ungesättigten Aldehyden umsetzen.

## Beispiel 1

### a) Katalysatorherstellung

Man löst 89,6 g Kupferpulver in 660 $cm^3$ 33%iger Salpetersäure und vermischt diese Lösung bei Raumtemperatur mit der Lösung von 83,4 g $PdCl_2$ in 400 $cm^3$ eines warmen Gemisches aus 66%iger Salpetersäure und 32%iger Salzsäure (Volumenverhältnis 1 : 1) sowie mit der Lösung von 6,25 g $TeO_2$ in 1000 $cm^3$ warmer, 16%iger Salzsäure. Man legt 1000 g Aktivkohle (0,3 bis 0,5 mm) vor, die zuvor bei 70°C 5 Stunden mit 15%iger Salpetersäure gerührt, nach dem Abnutschen neutral gewaschen und bei 150°C unter Vakuum getrocknet worden war und fügt die vereinigte Metallsalzlösung hinzu. Anschließend gibt man soviel Wasser hinzu, daß die Kohle völlig benetzt ist.

Dann wird am Rotationsverdampfer bei 85°C und unter Wasserstrahlvakuum zur Trockene eingedampft. Der Katalysator wird 2 Stunden bei 150°C im Vakuumtrockenschrank und dann 2 Stunden bei 150°C im Röhrenofen unter strömendem Stickstoff getrocknet. Anschließend leitet man bei 400°C zur Aktivierung des Katalysators bei Raumtemperatur mit Methanol gesättigten Stickstoff und schließlich bei 800°C 30 Minuten Wasserstoff (20 l/Stunde) über den Katalysator. Man läßt unter strömendem Stickstoff auf Raumtemperatur abkühlen.

Nach der Elementaranalyse enthält der Katalysator 5,24% Palladium, 8,8% Kupfer und 0,53% Tellur.

### b) 1,4-Diacetoxy-2,7-octadien durch Acetoxilierung von 1,3,7-Octatrien

Eine Apparatur, die aus einem 1-Liter-Dreihalskolben mit Anschützaufsatz, Tropftrichter, Begasungsrührer, Innenthermometer, Gaseinleitungsrohr und Rückflußkühler mit aufgesetztem Trockeneiskühler besteht, wird mit Stickstoff gespült. Dann gibt man eine Suspension von 25 g des nach Beispiel 1a) hergestellten Katalysators in 543 g Eisessig in die Apparatur. Man erhitzt auf 95°C, leitet im Verlauf von 4 Stunden 12 l Sauerstoff ein und tropft gleichzeitig 54 g 1,3,7-Octatrien zu. Man leitet dann weitere 30 Minuten bei 95°C insgesamt 1,5 l Sauerstoff ein. Anschließend durchspült man die Apparatur 30 Minuten mit Stickstoff. Man läßt abkühlen und entfernt den Katalysator durch Filtrieren. Die anfallende Essigsäurelösung (598 g) wird am Rotationsverdampfer eingedampft. Durch fraktionierte Destillation des Rückstandes erhält man neben 37 g unumgesetztem 1,3,7-Octatrien (Umsatz 32%) 23,1 g 1,4-Diacetoxy-2,7-octadien (65%, bezogen auf umgesetztes 1,3,7-Octatrien) vom Siedepunkt 101 bis 106°C/0,7 mbar, $n_D^{20}=1{,}4539$.

## Beispiel 2

### 2,7-Dimethyl-1,4-diacetoxy-2,7-octadien durch Acetoxilierung von 2,7-Dimethyl-1,3,7-octatrien

Man verfährt wie in Beispiel 1b) beschrieben, wobei man jedoch eine Suspension von 12,5 g eines nach der DE-OS 2 820 519 hergestellten Pd/Cu-Katalysators (5,27% Pd, 8,59% Cu) auf Aktivkohle als Träger in 500 g Essigsäure in die Apparatur gibt. Innerhalb von 2 Stunden werden bei 95°C 6 l

Sauerstoff eingeleitet und 34 g 2,7-Dimethyl-1,3,7-octatrien zugetropft. Man leitet dann weitere 30 Minuten lang bei 95°C insgesamt 1,5 l Sauerstoff ein. Anschließend durchspült man die Apparatur 30 Minuten mit Stickstoff. Nach der in Beispiel 1b beschriebenen Aufarbeitung und fraktionierten Destillation der Essigsäurelösung erhält man neben 25 g unumgesetztem 2,7-Dimethyl-1,3,7-octatrien (Umsatz 26%) 11 g 2,7-Dimethyl-1,4-diacetoxy-2,7-octadien vom Siedepunkt 113 bis 115°C/1,2 mbar, $n_D^{20}$=1,4625.

Beispiel 3

Herstellung von 2,6-Dimethyl-1,4-diacetoxy-2,7-octadien durch Acetoxilierung von 2,6-Dimethyl-1,3,7-octatrien:

In gleicher Weise wie in Beispiel 1b) beschrieben, werden in eine Suspension aus 12,5 g eines nach der DE-OS 2 217 452 hergestellten Pd/Te-Katalysators (5,1% Pd, 0,9% Te) auf Aktivkohle als Träger und 540 g Essigsäure innerhalb von 2 Stunden bei 95°C 6 l Sauerstoff eingeleitet und 34 g 2,6-Dimethyl-1,3,7-octatrien eingetropft. Dann leitet man weitere 30 Minuten lang bei 95°C insgesamt 1,5 l Sauerstoff ein. Anschließend durchspült man die Apparatur 30 Minuten mit Stickstoff. Man erhält nach Aufarbeitung und Destillation neben 21 g unumgesetztem 2,6-Dimethyl-1,3,7-octatrien (Umsatz 38%) 15,2 g 2,6-Dimethyl-1,4-diacetoxy-2,7-octadien vom Siedepunkt 94 bis 98°C/0,8 mbar, $n_D^{20}$=1,4576.

Beispiel 4

In gleicher Weise wie in Beispiel 1b) beschrieben, werden in eine Suspension von 25 g eines nach Beispiel 1a) hergestellten Pd/Cu/Te-Katalysators (5,04% Pd, 8,2% Cu, 0,55% Te) auf Aktivkohle als Träger in 543 g Essigsäure innerhalb von 4 Stunden bei 95°C 12 l Sauerstoff eingeleitet und 50 g 7-Methyl-3-methylen-1,6-octadien (Mycren) zugetropft. Man leitet dann weitere 30 Minuten lang bei 95°C insgesamt 1,5 l Sauerstoff ein; anschließend durchspült man die Apparatur 30 Minuten lang mit Stickstoff. Nach der in Beispiel 1b) beschriebenen Aufarbeitung und fraktionierten Destillation der Essigsäurelösung erhält man neben 18 g unumgesetztem 7-Methyl-3-methylen-1,6-octadien (Umsatz 64%) 37 g 2-(4-Methyl-3-pentenyl)-1,4-diacetoxy-2-buten vom Siedepunkt 112 bis 119°C/0,5 mbar, $n_D^{20}$=1,4678.

**Patentansprüche**

1. Diacyloxyalkadiene der Formel

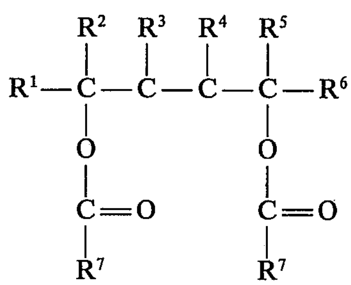

(I)

in der die Reste $R^1$ bis $R^5$ Wasserstoffatome oder Kohlenwasserstoffreste mit 1 bis 6 C-Atomen, $R^6$ ein Wasserstoffatom oder einen Kohlenwasserstoffrest mit 3 bis 9 C-Atomen und mindestens einer nicht-konjugierten Doppelbindung und $R^7$ ein Wasserstoffatom oder einen Alkylrest mit 1 bis 5 C-Atomen bedeuten, und für den Fall, daß $R^6$ ein Wasserstoffatom bedeutet, mindestens einer der Reste $R^1$ bis $R^5$ für einen $R^8-CH_2$-Rest steht, in dem $R^8$ einen Kohlenwasserstoffrest mit 2 bis 5 C-Atomen bedeutet, der mindestens eine nicht-konjugierte Doppelbindung enthält.

2. Verfahren zur Herstellung der Diacyloxyalkadiene nach Anspruch 1, dadurch gekennzeichnet, daß man aliphatische Triene der Formel

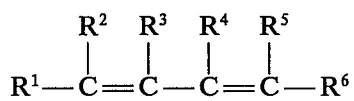

(II)

in der die Reste $R^1$ bis $R^6$ die im Anspruch 1 genannte Bedeutung haben, in Gegenwart von Trägerkatalysatoren, die Palladium oder Platin, und außerdem noch Kupfer und/oder Tellur enthalten, und von Sauerstoff mit Carbonsäuren der Formel $R^7-COOH$, in der $R^7$ die im Anspruch 1 genannte Bedeutung hat, bei Temperaturen von 70 bis 180° C behandelt.

## Claims

1. A diacyloxyalkadiene of the formula

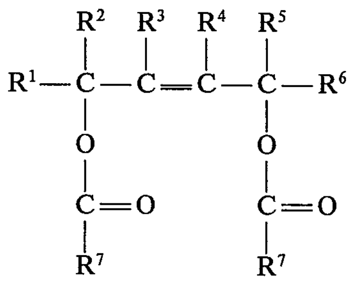

(I)

where $R^1$ to $R^5$ are each hydrogen or a hydrocarbon radical of 1 to 6 carbon atoms, $R^6$ is hydrogen or a hydrocarbon radical of 3 to 9 carbon atoms having one or more non-conjugated double bonds, and $R^7$ is hydrogen or alkyl of 1 to 5 carbon atoms, and in the case in which $R^6$ is hydrogen, one or more of $R^1$ to $R^5$ are $R^8-CH_2-$, where $R^8$ is a hydrocarbon radical of 2 to 5 carbon atoms which contains one or more non-conjugated double bonds.

2. A process for the preparation of a diacyloxyalkadiene as claimed in claim 1, wherein an aliphatic triene of the formula

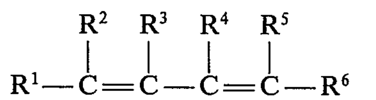

(II)

where $R^1$ to $R^6$ have the meanings given in claim 1, is treated at a temperature of from 70 to 180° C with a carboxylic acid of the formula $R^7-COOH$, where $R^7$ has the meaning given in claim 1, in the presence of a supported catalyst which contains palladium, or platinum as well as copper and/or tellurium, and in the presence of oxygen.

## Revendications

1. Diacyloxy-alcadiènes de la formule

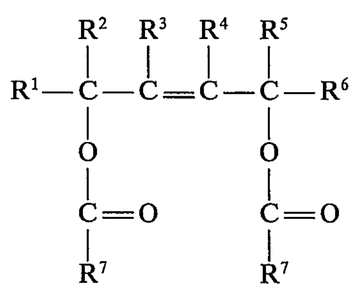

(I)

dans laquelle les substituants $R^1$ à $R^5$ désignent des atomes d'hydrogène ou des radicaux hydrocarbonés en $C_1$ à $C_6$, $R^6$ représente un atome d'hydrogène ou un radical hydrocarboné en $C_3$ à $C_9$, comportant au moins une double liaison non conjuguée, et $R^7$ désigne un atome d'hydrogène ou un radical alkyle en $C_1$ à $C_5$, et quand $R^6=H$, un des substituants $R^1$ à $R^5$ au moins représente un groupe $R^8-CH_2-$, où $R^8$ désigne un radical hydrocarboné en $C_2$ à $C_5$, comportant au moins une double liaison non conjugée.

2. Procédé de préparation de diacyloxy-alcadiènes suivant la revendication 1, caractérisé en ce que l'on traite des triènes aliphatiques de la formule

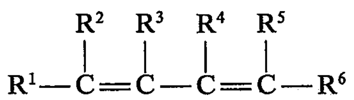

(II)

dans laquelle les substituants $R^1$ à $R^6$ possèdent la signification définie dans la revendication 1, à des températures de 70 à 180°C, en présence de catalyseurs sur support, contenant du palladium ou du platine, ainsi que du cuivre et(ou) du tellure, et d'oxygène avec des acides carboxyliques de la formule $R^7$—COOH, dans laquelle $R^7$ possède la signification définie dans la revendication 1.